(19) Europäisches Patentamt
European Patent Office
Office européen des brevets

(11) EP 0 922 670 B1

(12) # EUROPÄISCHE PATENTSCHRIFT

(45) Veröffentlichungstag und Bekanntmachung des Hinweises auf die Patenterteilung:
**02.04.2003 Patentblatt 2003/14**

(51) Int Cl.[7]: **C01B 17/74**

(21) Anmeldenummer: **98122921.4**

(22) Anmeldetag: **02.12.1998**

(54) **Verfahren zur Rückgewinnung von Schwefelsäure aus schwefelhaltigen Nebenprodukten eines Prozesses zur Herstellung von 2-Hydroxy-4-methylthiobuttersäure (MHA)**

Process for recovering sulphuric acid from sulphur containing by-products of a process for preparing 2-hydroxy-4-methylthiobutyric acid (MHA)

Procédé de récupération d' acide sulfurique des sous-produits soufrés d' un procédé de préparation d'acide 2- hydroxy-4 -methylthiobutyrique (MHA)

(84) Benannte Vertragsstaaten:
**AT BE CH DE ES FR GB IT LI NL**

(30) Priorität: **09.12.1997 DE 19754562**

(43) Veröffentlichungstag der Anmeldung:
**16.06.1999 Patentblatt 1999/24**

(73) Patentinhaber: **Degussa AG**
**40474 Düsseldorf (DE)**

(72) Erfinder:
- **Hasseberg, Hans-Albrecht Dr.**
  **63584 Gründau-Lieblos (DE)**
- **Hasselbach, Hans-Joachim Dr.**
  **63571 Gelnhausen (DE)**
- **Huthmacher, Klaus Dr.**
  **63571 Gelnhausen (DE)**
- **Häfner, Volker**
  **63505 Langenselbold (DE)**
- **Heinzel, Harald**
  **60488 Frankfurt (DE)**

(56) Entgegenhaltungen:
**DE-A- 2 148 244**
**DE-A- 4 036 899**
**US-A- 5 595 713**

- **DATABASE WPI Section Ch, Week 8431 Derwent Publications Ltd., London, GB; Class E36, AN 84-192166 XP002095279 & JP 59 108995 A (HITACHI ZOSEN CORP) , 23. Juni 1984**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 922 670 B1

## Beschreibung

Die Erfindung betrifft ein Verfahren zur Rückgewinnung von Schwefelsäure aus Sulfat-haltigen Nebenprodukten, die in Prozessen zur Herstellung von 2-Hydroxy-4-methylthiobuttersäure (MHA) entstehen, wobei die Schwefelsäure in einer Qualität anfällt, in der sie in solchen Prozessen direkt wiedereingesetzt werden kann.

MHA ist das Hydroxyanaloge der essentiellen Aminosäure Methionin in racemischer Form und kann als Zusatzstoff in Futtermitteln, insbesondere für die Geflügelzucht,aber auch in vielen anderen Bereichen, vor allem in Form seiner wäßrigen Konzentrate eingesetzt werden.

Der technisch ausschließlich verwendete Syntheseweg geht aus von Methylmercaptopropionaldehyd (MMP), der durch HCN-Addition in das entsprechende Cyanhydrin (MMP-CH) überführt wird, welches dann schwefelsauer katalysiert zunächst zum MHA-Amid und in einem weiteren Schritt zur Hydroxysäure MHA hydrolysiert wird, wobei die Schwefelsäure in Ammoniumhydrogensulfat und gegebenfalls Ammoniumsulfat überführt wird.

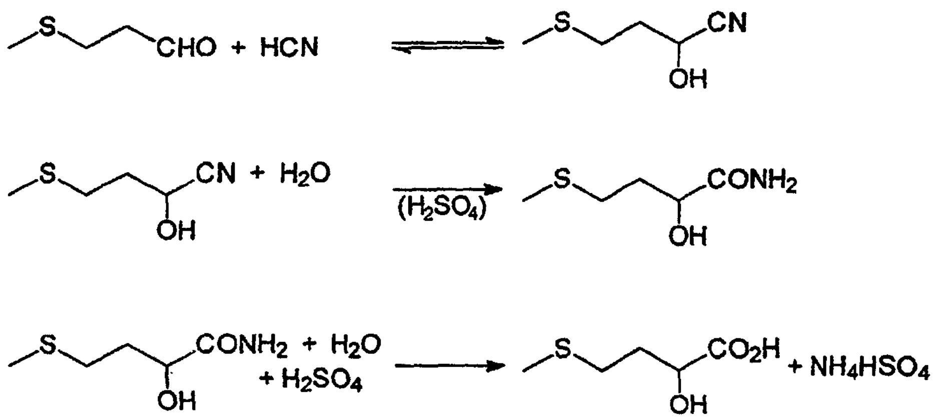


(MMP-CH)

(MHA-Amid)

(MHA)

Ausgehend vom MHA-Hydrolysat, welches somit neben entsprechenden Anteilen an Wasser das Ammoniumhydrogensulfat/-sulfat enthält, gibt es verschiedene Verfahren, um MHA zu isolieren, welche in der deutschen DE-OS 19524054 bzw.in der DE-PS 4428608 zusammenfassend beschrieben werden.

Diese Verfahren beinhalten entweder eine Lösungsmittelextraktion oder auch Fällungsschritte oder eine Kombination aus beiden zur MHA-Abtrennung vom mitentstandenen Salz.

Bei jedem dieser Prozesse wird neben dem Produkt MHA noch ein der zuvor eingebrachten Schwefelsäuremenge entsprechender Anteil an Ammoniumhydrogensulfat/-sulfat in Form einer Lösung oder in einer mehr oder weniger reinen

Feststoffqualität erzeugt. Da MHA in einer Größenordnung von ca. 20000 bis 300000 jato pro Anlage hergestellt wird, fallen auch in etwa der gleichen Größenordnung Sulfatsalzmengen an, welche bisher teuer entsorgt bzw. deponiert werden müssen. Insbesondere das Deponieren solch gigantischer Salzmengen ist unter ökologischen Gesichtspunkten jedoch nicht vertretbar bzw. durch weiter steigende Gebühren auch nicht ökonomisch sinnvoll. Zur Verwertung der Abfallsalze ist es daher besonders wünschenswert, aus den Sulfaten Schwefelsäure zurückzugewinnen und diese in den MHA-Prozeß zurückzuführen.

Ein ganz ähnlicher Verfahrensverbund ist bereits aus dem technischen Prozeß zur Herstellung von Methylmethacrylat (MMA) bekannt, wie es z.B. im US-Patent 3549320 beschrieben wird. Hier wird Acetoncyanhydrin zunächst mit Schwefelsäure zu Methacrylamid-Sulfat und anschließend mit Methanol zu Methylmethacrylat und Ammoniumbisulfat umgesetzt. Nach Abtrennung des MMA wird der Schwefelsäure und Ammoniumbisulfat enthaltende Rückstand in einer Spaltkontaktanlage (SK) in Schwefelsäure umgewandelt. Dieses aus dem Stand der Technik bekannten Verfahren wird im US-Patent 5498790 zur Abfallsalzverwertung im MHA-Prozeß vorgeschlagen. Dabei wird zunächst die hauptsächlich Ammoniumbisulfat bzw. Ammoniumsulfat, Wasser und geringe organische Nebenprodukte enthaltende Raffinatlösung aus der MHA-Extraktion in einem Spaltofen zusammen mit einem Brennstoff bei ca. 900 - 1200 °C zu $SO_2$, $N_2$, $O_2$, $CO_2$ und $H_2O$ verbrannt:

$$2\ NH_4HSO_4 + 1/2\ O_2 \rightarrow N_2 + 2\ SO_2 + 5\ H_2O$$

Das heiße $SO_2$-haltige Verbrennungsgas wird nach Abkühlung auf 35- 45 °C zur Auskondensation von Wasseranteilen und nachfolgendem Wiederaufheizen zusammen mit Sauerstoff in einen Kontaktofen geführt und dort das $SO_2$ am $V_2O_5$-Kontakt bei Temperaturen von mindestens 420 ° C zu $SO_3$ oxidiert:

$$SO_2 + 1/2\ O_2 \rightarrow SO_3.$$

Die wirtschaftliche Durchführung dieses Verfahrens ist aber an $SO_2$-Mindestkonzentrationen gebunden.

Das Schwefeltrioxid-haltige Kontaktgas wird nach altbekannter Verfahrensweise in Oleum absorbiert und durch nachfolgende Verdünnung mit Wasser Schwefelsäure mit einer für den MHA-Prozeß benötigten Konzentration von ca. 65 Gew% hergestellt.

Die Nachteile dieses in der US-PS 5,498,790 beschriebenen Verfahrens liegen in der aufwendigen und komplizierten Verfahrenstechnik für den Kontaktofen, bei der der $SO_2$haltige Spaltgasstrom mit Sauerstoff über mehrere Kontakthorden geführt werden muß und nach einer Zwischenabsorption des bereits gebildeten $SO_3$ mit Zwischenkühlung und Wiederaufheizen auf die Reaktionstemperatur der Umsatz des restlichen Schwefeldioxids auf der letzten Horde bewerkstelligt wird.

Weitere Nachteile sind die hohe Reaktionstemperatur von über 420 ° C,die zudem durch Wiederanheizen des auf 35 - 45 °C abgekühlten $SO_2$-Verbrennungsgases erreicht wird, sowie der nicht quantitative Umsatz des $SO_2$-Anteils bei der katalytischen Oxidation im Kontaktofen, der zu einem Rest $SO_2$-Gehalt im Abgas nach dem Kontaktofen führt. Dieser Anteil muß durch eine geeignete Nachbehandlung auf die erlaubten gesetzlichen Grenzwerte abgesenkt werden. Das in der US-PS 5,498,790 beschriebene Verfahren arbeitet bevorzugt mit einer Mindestkonzentration von 70 Gew.-% Sulfatsalz im Eingangsstrom des Verbrennungsofens, da eine geringere Konzentration zu einem Mehrbedarf an Brennstoff für die Verdampfung des hohen Wasseranteils und zu höheren Inertgasanteilen an $CO_2$, $N_2$ etc. führt. In diesem Fall sinkt die $SO_2$-Konzentration so stark ab, daß der Kontaktofen nicht mehr effektiv betrieben werden kann. Insbesondere Ammonium-haltige Lösungen nach US-PS 4,912,257, deren maximale Sulfatkonzentration deutlich unter 70 Gew.-% liegt, können erst nach weiterer Aufkonzentration als Suspension eingeführt werden. Dies führt im Betrieb zu größeren Schwierigkeiten, wie z. B. Verstopfungen und ist somit wenig praktikabel (vergleiche US 5,498,790, Col. 14 unten bzw. 15 oben).

Weitere Nachteile bestehen in der Verwendung eines an ausreichend hohe $SO_2$-Konzentrationen gebundenen Schwermetallkatalysators und der sich daraus ergebenden Gefahr der Produktkontamination mit Katalysatorresten sowie mit $NO_x$, das durch katalytische Oxidation bei den genannten Reaktionstemperaturen gebildet werden kann. Diese Verunreinigungen können bei Rückführung der Schwefelsäure in den MHA-Prozeß in das als Futtermitteladditiv eingesetzte MHA-Endprodukt gelangen. Dort sind sie naturgemäß unerwünscht.

Aufgabe der Erfindung ist es, ein einfacheres Verfahren zur Wiedergewinnung von Schwefelsäure aus Sulfathaltigen Nebenprodukten der MHA-Herstellung zur Verfügung zu stellen, mit dessen Hilfe man unter möglichst milden Bedingungen und ohne Verunreinigung durch Schwermetalle und $NO_x$ eine Schwefelsäurequalität erhält, die direkt in der Hydrolysestufe eines MHA-Herstellungsprozesses wiedereingesetzt werden kann. Das Verfahren soll darüberhinaus unabhängig von der $SO_2$-Konzentration der Verbrennungsgase einsetzbar sein und ein Abgas nach der Schwefelsäureerzeugung liefern, das ohne weitere Nachbehandlung in die Atmosphäre abgegeben werden kann.

Gegenstand der Erfindung ist ein Verfahren zur Wiedergewinnung von Schwefelsäure aus bei der schwefelsauren Hydrolyse von MMP-Cyanhydrin anfallenden Sulfat-haltigen Lösungen oder Feststoffe, wobei die Sulfate in einem Verbrennungsofen zu $SO_2$ umgesetzt werden, das dadurch gekennzeichnet ist, daß das $SO_2$-haltige Gasgemisch durch eine wäßrige schwefelsaure Lösung von $H_2O_2$ geleitet und in Schwefelsäure überführt wird, gemäß der Bruttogleichung;

$$SO_2 + H_2O_2 \rightarrow H_2SO_4$$

Dabei gelingt es, Schwefelsäure in einer Konzentration und in einer Qualität herzustellen, die direkt zur Herstellung von MHA durch Hydrolyse von MMP-Cyanhydrin eingesetzt werden kann.

In der erfindungsgemäß hergestellten Schwefelsäure findet sich keine nachweisbare $NO_x$-Verunreinigung, so daß diese Schwefelsäure ohne Bedenken für den Hydrolyseschritt eingesetzt werden kann.

Man geht erfindungsgemäß bevorzugt so vor, daß man das $SO_2$haltige Verbrennungsgas bei einer Temperatur zwischen 0 und 100 ° C, vorzugsweise zwischen 10 und 80 ° C, insbesondere 20 bis 60 ° C durch eine Füllkörperkolonne leitet, in der sich eine saure wäßrige Lösung von Wasserstoffperoxid befindet, das im Kreislauf gefahren wird. Vorzugsweise setzt man Wasserstoffperoxid mit einer Konzentration zwischen 10 und 90 Gew.-%, vorzugsweise zwischen 20 und 80 Gew.-%, insbesondere zwischen 30 und 60 Gew.-% ein, mit der Maßgabe, daß verbrauchtes Wasserstoffperoxid entsprechend den $SO_2$-Mengen in der gewünschten bzw. notwendigen Konzentration nachdosiert wird.

Für den Fachmann selbstverständlich, wird das $SO_2$-haltige Gas gleichzeitig in dieser Einrichtung und/oder vor Eintritt in diese Einrichtung abgekühlt.

In einer üblichen Arbeitsweise setzt man zur Absorption des Schwefeldioxids eine einfache Füllkörperkolonne ein.

Diese wird im allgemeinen als gekühlter Absorptionskreislauf gefahren.
Als bevorzugte Ausführungsform gilt jedoch die zumindest zweistufige Absorption in einer der für diese Zwecke bekannten Absorptionskolonnen.

Vorteilhaft geht man so vor, daß das $SO_2$-haltige Verbrennungsgas in der Nähe des Sumpfes in die Kolonne eingeführt wird. Die Kolonnenfüllung wird gegebenenfalls mehrfach umgewälzt.
In einer bevorzugten Ausführungsform bringt man die Kreislaufflüssigkeit solange mit dem $SO_2$-haltigen Gas in Kontakt, bis in der Kreislauflösung praktisch kein $H_2O_2$ mehr vorhanden ist.
Die gewünschte $H_2SO_4$-Konzentration wird über die $H_2O_2$-Konzentration der eingesetzten Wasserstoffperoxidlösung eingestellt.
Diese liegt zwischen 10 und 90 Gew.-%, insbesondere 20 und 80 Gew.-%, besonders bevorzugt zwischen 30 und 60 Gew.-%.

Erfindungsgemäß arbeitet man so, daß man eine Schwefelsäurekonzentration von 50 bis 78 Gew.-% erzielt.

In einer vorteilhaften Ausführungsform trägt man die so erzeugte Schwefelsäure kontinuierlich aus dem gegebenenfalls mehrstufig ausgelegten Reaktor aus.

Je nach $SO_2$-Konzentration im Verbrennungsgas wird bei gegebenem Volumenstrom des Verbrennungsgases dabei unterschiedlich viel $H_2SO_4$ pro Zeiteinheit erzeugt.

In einer technischen Ausführungsform empfiehlt es sich, eine zweistufige Apparatur einzusetzen, die aus 2 Absorptionskreisen besteht (vgl. Figur 2), wobei der 1. Absorptionskreis mit einem molaren $H_2O_2$-Unterschuß bezüglich des $SO_2$-Anteils im dort eingeleiteten Verbrennungsgas und der 2. Absorptionskreis mit einem $H_2O_2$-Überschuß betrieben wird. Der aus dem 1. Kreis ausgetragene teiloxidierte Gasstrom wird dem 2. Kreis zugeführt und dort der $SO_2$-Rest umgesetzt. Dazu wird der 2. Kreis mit frischem $H_2O_2$ im molaren Überschuß bezogen auf den dort eingespeisten $SO_2$-Anteil beaufschlagt. Die im 2. Kreis befindliche Schwefelsäure mit $H_2O_2$-Restgehalt wird kontinuierlich zur Beaufschlagung des 1. Kreises ausgetragen und dort das restliche $H_2O_2$ aus dem 2. Kreis vollständig mit dem Rohgas umgesetzt. Aus dem 1.Kreis wird kontinuierlich $H_2O_2$-freie Schwefelsäure mit der entsprechend eingestellten Konzentration als Recyclierungsprodukt erhalten.

Ein gegebenenfalls in der so erzeugten Schwefelsäure enthaltener geringfügiger $H_2SO_3$-Anteil aus unumgesetzten $SO_2$ ist für den Einsatzzweck der Hydrolyse von MMP-Cyanhyrin zu MHA unkritisch, weil dadurch keinerlei Nebenreaktionen verursacht werden können.

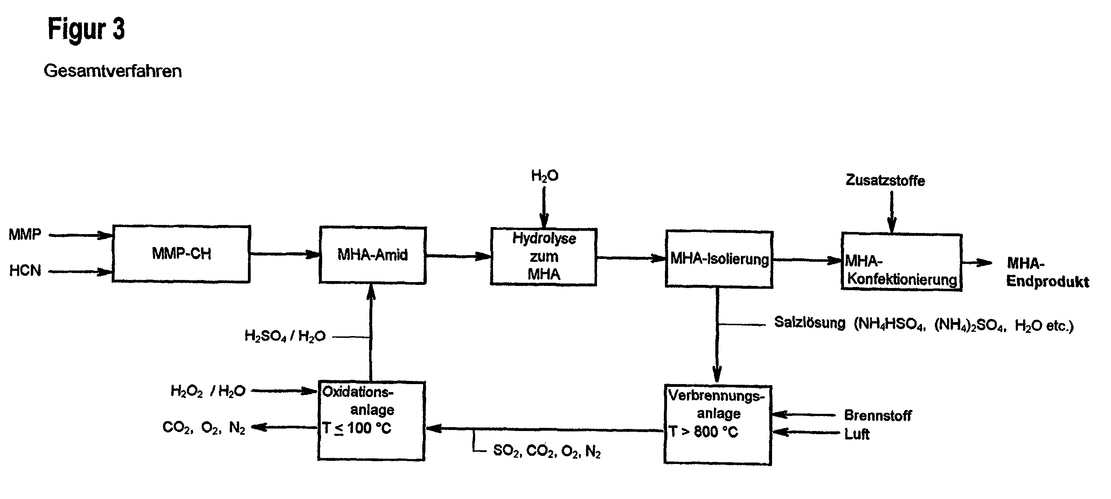

Besonders elegant läßt sich eine solche $H_2SO_4$-Regenerierung und Recyclierung innerhalb eines entsprechenden Kreisprozesses realisieren, bei dem die Schwefelsäure in die Stufe der schwefelsauren Hydrolyse von MMP-CH zu MHA-Amid zurückgeführt werden kann (vgl. Figur 3).

Darüberhinaus ist es ohne weiteres möglich, zur Erzeugung von $SO_2$ im Verbrennungsofen neben den Sulfaten aus einem MHA-Prozeß zusätzliche Schwefelquellen zu verwenden. Zum einen kann direkt elementarer Schwefel verbrannt werden, was insbesondere bei erhöhten Schwefelsäurebedarf sinnvoll ist. Zum anderen können aber auch Sulfate aus anderen Prozessen mitverwertet werden. Hier ist insbesondere an $NH_4HSO_4$- bzw. $(NH_4)SO_4$-haltige Waschlösungen z.B. aus einem Blausäureprozeß zu denken. Dort wird bei der Erzeugung von HCN aus $CH_4$ und $NH_3$ das restliche $NH_3$ in Wäschern mit wäßriger Schwefelsäure ausgewaschen. Die auf diese Weise erzeugten Sulfathaltigen Lösungen können unabhängig von ihrer Konzentration in einem Verbrennungsofen direkt mitverwertet werden. In der nachfolgenden $H_2O_2$-Oxidationsstufe wird daraus wieder Schwefelsäure erzeugt, die in den HCN-Prozeß zurückgeführt werden kann.

Da HCN zur MMP-Cyanhydrinherstellung gebraucht wird, und eine entsprechende HCN-Anlage aufgrund der aus Sicherheitsgründen nur beschränkt möglichen Blausäuretransporte in aller Regel in unmittelbarer Nähe einer MHA-Anlage stehen muß, ist auch hier ein direkter Prozeßverbund möglich. Dieser ist aus ökonomischen sowie modernen sicherheitstechnischen als auch ökologischen Gesichtspunkten außerordentlich vorteilhaft.

Auch geruchsintensive schwefelhaltige Abwässer oder Abgase, wie sie neben Sulfat in einem MHA-Prozess typischerweise anfallen, die aber auch aus anderen Anlagen vorzugsweise am gleichen Standort stammen können und/oder organische Abfälle aus einem MHA-Prozeß bzw. anderen Prozessen, sind im Verbrennungsofen mitzuverwerten.

Hier ist es im Gegensatz zur in US-PS 5,498,790 beschriebenen katalytischen Luft-Oxidation von $SO_2$-haltigem Verbrennungsgas bei > 420°C völlig unerheblich, wieviel Gasballast durch erhöhte Inertanteile oder Brennstoffmehrverbrauch bei Verbrennung von verdünnten Lösungen miterzeugt wird. In der erfindungsgemäß verwendeten $H_2O_2$-Oxidation eines solchen Verbrennungsgases können auch beliebig niedrige $SO_2$-Konzentrationen noch quantitativ mit Wasserstoffperoxid zu Schwefelsäure umgewandelt werden, wie aus entsprechenden Verfahren zur Abgasentschwefelung bekannt ist. Eine Mindestanforderung für die Sulfat- bzw die Schwefelkonzentration im Eingangsstrom

zum Verbrennungsofen besteht im Gegensatz zum Verfahren nach der US-PS 5,498,790 nicht.
Das auf <50 ° C abgekühlte $SO_2$-haltige Verbrennungsgas kann außerdem direkt ohne Wiederaufheizen der $H_2O_2$-Oxidationsstufe zugeführt werden. Eine Abtrennung von bereits anteilig vorhandenem $SO_3$, wie es beim katalytischen Prozeß notwendig ist, entfällt hier ebenfalls.

Das beanspruchte Verfahren zeichnet sich insbesondere dadurch aus, daß es Abfallströme vermeidet, und die zur Hydrolyse eingesetzte Schwefelsäure vollständig zurückgewonnen werden kann. Selbst MHA-haltige Fehlchargen können als Brennstoff in den Verbrennungsofen eingeführt werden. Der darin enthaltene Schwefel wird in Schwefeldioxid überführt, das erfindungsgemäß mit $H_2O_2$ in Schwefelsäure umgesetzt wird.

Folgende präparativen **Beispiele** verdeutlichen den Gegenstand der Erfindung weiter:

Analytische Bestimmungsmethoden und Definitionen

Die Gehalte an MHA-Monomerem bzw. von MHA-Amid wurden in den Prozeßlösungen quantitativ per HPLC durch Vergleich mit einem externen Standard (Reinsubstanz) bestimmt.

Der Gehalt an MHA-Gesamt (MHAges) =MHA-Monomeres + MHA-(Dimere + Oligomere) + MHA-Amid (ggf.) wurde durch titrimetrische Bestimmung der Thioether-Funktion mit $KBr/KBrO_3$-Maßlösung und als Summe der entsprechenden MHA-Monomerequivalente in [Gew%] bzw. [g] bzw. [mol] bzw. [Mol%] ausgedrückt.

Der Gehalt an MHA-Dimeren + MHA-Oligomeren (DIM + OLI) wurde durch Berechnung der Differenz aus MHA-Gesamt und MHA-Monomer + MHA-Amid (gegebenenfalls) ermittelt und als Summe der entsprechenden MHA-Monomerequivalente in [Gew%] bzw. [g] bzw. [mol] bzw. [Mol%] ausgedrückt.

Der Schwefelsäuregehalt wurde durch alkalimetrische Titration, der $H_2O_2$-Gehalt durch einen Merck-Schnelltest bestimmt.

Der Wassergehalt wurde per Titration nach Karl-Fischer, der Nitrat-, Sulfit-, Sulfat- bzw. Ammonium-Gehalt per Ionenchromatographie nach Standard-Verfahren bestimmt.

Verfahrensbeschreibung unter Bezugnahme auf Figur 1

In Figur 1 ist der schematische Aufbau der für Beispiel 1 verwendeten Apparatur gezeigt. Diese besteht im wesentliche aus folgenden Apparaten

| | |
|---|---|
| Vorratsbehälter für Wasserstoffperoxid | B 1 |
| Auffangbehälter für gebildete Schwefelsäure | B 2 |
| Dosierpumpe für Wasserstoffperoxid | P 1 |
| Kreislaufpumpe | P 2 |
| Glas-Absorptionskolonne mit Füllkörpern | K 1 |
| Wärmeaustauscher zur Kühlung | W 1 |

Das Gasgemisch, das in seiner Zusammensetzung dem typischerweise aus der Verbrennung resultierenden Rohgas entsprach, wurde mit einer Temperatur von 20 - 25 ° C in den unteren Teil einer mit Füllkörpern gefüllten Absorptionskolonne K 1 geleitet. Als Absorptionslösung diente die gebildete wäßrige Schwefelsäure die mit der Kreislaufpumpe P 2 über den Kopf der Kolonne im Kreis gefahren wurde. Die bei der Reaktion entstehende Energie wurde mit dem Wärmeaustauscher W 1 abgefangen und so die Temperatur im Umlauf auf max. 3 ° C über der Gaseintrittstemperatur begrenzt. Das für die Reaktion notwendige Wasserstoffperoxid (50 Gew.% $H_2O_2$) wurde aus dem Vorratsbehälter B 1 mit der Dosierpumpe P 1 in den Kreislauf gepumpt. Der Überschuß $H_2O_2$ in der Absorptionsflüssigkeit betrug max. 10 Mol % bezogen auf den eingeleiteten $SO_2$-Strom. Die gebildete Schwefelsäure wurde im Seitenstrom in den Auffangbehälter B 2 abgelassen.

**Beispiel 1**

Die Absorptionskolonne wurde mit gerade soviel Wasser gefüllt, daß der Kühl-und Absorptionskreislauf sicher gefahren werden konnte. In die Kolonne wurden 100 l/h eines Gasgemisches der nachfolgenden Zusammensetzung eingeleitet. Die Zusammensetzung entspricht der eines typischen Verbrennungsgases aus einem Verbrennungsofen zur Erzeugung von $SO_2$ aus Sulfat-haltigem Raffinat eines MHA-Prozesses:

| | | |
|---|---|---|
| 82 Vol.% | (3,33 mol/h) | $N_2$ |

(fortgesetzt)

| | | |
|---|---|---|
| 7 Vol.% | (0,28 mol/h) | $CO_2$ |
| 5 Vol.% | (0.20 mol/h) | $SO_2$ |
| 6 Vol.% | (0,24 mol/h) | $O_2$ |

Die Zugabe des 50 Gew.-% $H_2O_2$( 13,6g/h, 0,2 mol/h ) erfolgte mit geringem Vorlauf im stöchiometrischen Verhältnis. Die Gaseintrittstemperatur betrug 20 ° C. Die Temperatur im Kühlkreislauf stieg nicht über 23 ° C. Im Abstand von mehreren Stunden wurde der $H_2SO_4$-Gehalt in der abfließenden Schwefelsäure bestimmt. Die $H_2SO_4$-Konzentration stieg dabei bis auf 74,7 Gew-% an. Ein Verlust an Wasserstoffperoxid konnte nicht nachgewiesen werden. Es wurden durchschnittlich 26 g/h (0,2 mol/h) einer max.75% Gew.-% $H_2SO_4$ im Auffangbehälter B2 erhalten. Die erzeugte Schwefelsäure hatte folgende Zusammensetzung:

| | |
|---|---|
| Gehalt $H_2SO_4$ | 74,7 Gew.% |
| Gehalt $H_2O$ | 24,85 Gew.% |
| Gehalt $SO_3^{2-}$ | 1570 mg/kg |
| Gehalt $H_2O_2$ | <0,5mg/l |
| Gehalt $NO_3^-$ | < 10mg/kg |

**Beispiel 2**

In der Absorptionsapparatur wurde 306 g 50 %iges Wasserstoffperoxid (4,5 mol) vorgelegt und im Kreis gepumpt. Bei einer max. Temperatur von 23 ° C wurde die Gasmischung analog Beispiel 1 für mehrere Stunden eingeleitet. Es resultierten 596 g einer Schwefelsäure mit einem Gehalt von 74.0 Gew.-% (4,5 mol). Ein Verlust an Wasserstoffperoxid konnte nicht nachgewiesen werden.

**Beispiel 3**

In einem Reaktionsbehälter mit Rührer Innenthermometer und Rückflußkühler wurden 236 g 74,7 Gew.-% $H_2SO_4$ (1,8 mol ) aus Beispiel 1 vorgelegt. Innerhalb von 30 Min. wurden 403 g (3,0 mol) 97,7 Gew.-% MMP-Cyanhydrin in den gerührten Reaktor dosiert. Die Reaktionstemperatur wurde während des Zulaufs und der anschließenden Nachreaktionszeit von 30 min auf 50 ° C gehalten. Mit beendeter Nachreaktion wurde das Reaktionsgemisch mit 540 g Wasser verdünnt und sofort in einen 21 Büchi-Druckreaktor mit Rührer und Innentemperaturmessung überführt. Die Reaktionslösung hatte einen Gehalt von 22,8 Gew.-% MHA-Amid und 15,3 Gew.-% MHA und wurde unter Rühren auf 120 ° C aufgeheizt und 3 Stunden bei dieser Temperatur weitergerührt. Nach Abkühlen auf Raumtemperatur wurden 1176 g MHA-Hydrolysat mit folgender Zusammensetzung isoliert:

| | | |
|---|---|---|
| 0,33 Gew.% | ( 0,9 % d.Th) | MHA-Amid |
| 36,26 Gew.% | (94,6 % d.Th) | MHA |
| 1,71 Gew.% | (ca.4,5 % d.Th) | MHA-Dim+Oli |
| 38,30 Gew.% | (100 % d.Th) | MHAges. |

Es wurden 256 g (0,663 mol MHA) des MHA-Hydrolysates mit 2mal 100 ml Methyl-tert.butylether extrahiert, die organischen Phasen vereinigt und im Wasserstrahlvakuum eingedampft. Das primär erzeugte MHA-Hochkonzentrat (100 g) wurde mit 12 g Wasser verdünnt und analysiert:

| | | |
|---|---|---|
| MHAges | 87,7 Gew% | 100 Mol% |
| MHA-Monomer | 77,2 Gew% | 88,0 Mol% |
| MHA-Dim+Oli | 10,5 Gew% | 12,0 Mol% |
| $H_2O$ | 12,0 Gew% | |
| $SO_4^{2-}$ | 0,2 Gew% | |
| $SO_3^{2-}$ | <0,1 Gew% | |
| $NO_3^-$ | < 10 ppm | |

## Patentansprüche

1. Verfahren zur Rückgewinnung von Schwefelsäure aus bei der schwefelsauren Hydrolyse von MMP-Cyanhydrin in Lösung oder fest anfallenden Sulfatsalzen, wobei die Sulfate in einem Verbrennungsofen zu $SO_2$ umgesetzt werden,
   **dadurch gekennzeichnet,**
   **daß** das $SO_2$-haltige Gasgemisch durch eine wäßrige schwefelsaure Lösung von $H_2O_2$ geleitet und das $SO_2$ in Schwefelsäure überführt wird und daß die Konzentration des Wasserstoffperoxids und des $SO_2$ so gewählt werden, daß man eine 50 bis 78%ige, vorzugsweise eine 60 bis 75%ige wäßrige Schwefelsäure erhält.

2. Verfahren nach Anspruch 1,
   **dadurch gekennzeichnet,**
   **daß** die Konzentration des eingesetzten Wasserstoffperoxids zwischen 10 und 90 Gew.-%, vorzugsweise zwischen 20 und 80 Gew.-%, besonders bevorzugt zwischen 30 und 60 Gew.-% liegt.

3. Verfahren nach Anspruch 1,
   **dadurch gekennzeichnet,**
   **daß** die Temperatur bei der $SO_2$-Oxidation zwischen 0 und 100 ° C, vorzugsweise zwischen 10 und 80 ° C, besonders bevorzugt zwischen 20 und 60 ° C liegt.

4. Verfahren nach Anspruch 1,
   **dadurch gekennzeichnet,**
   **daß** die Oxidation von $SO_2$ mit $H_2O_2$ wahlweise in einer ein-, zwei- oder mehrstufigen Apparatur durchgeführt wird.

5. Verfahren nach Anspruch 1,
   **dadurch gekennzeichnet,**
   **daß** neben den Sulfaten aus einem MHA-Prozeß Sulfatanteile aus anderen Quellen und/oder Schwefel-haltige Abgase bzw. Abwässer aus einem MHA-Prozeß bzw. anderen Prozessen und /oder organische Abfälle aus einem MHA-Prozeß bzw. anderen Prozessen dem Verbrennungsofen zugeführt werden.

6. Verfahren zur Herstellung von 2-Hydroxy-4-methylthiobuttersäure (MHA) durch Hydrolyse von MMP-Cyanhydrin mit Schwefelsäure zu einem Gemisch aus den Hauptkomponenten MHA und Ammoniumhydrogensulfat und/oder Ammoniumsulfat, Isolierung des MHA, Wiedergewinnung der Schwefelsäure aus den abgetrennten Sulfat-haltigen Mischungen und Wiederverwendung der so erzeugten Schwefelsäure zur Hydrolyse des MMP-Cyanhydrins
   **dadurch gekennzeichnet,**
   **daß** die abgetrennten Sulfat-haltigen Mischungen, vorzugsweise in Form einer wäßrigen Lösung in einem Verbrennungsofen bei über 800 ° C zu $SO_2$ umgesetzt werden, und das dabei erzeugte $SO_2$-haltige Gasgemisch durch Oxidation mit $H_2O_2$ in wäßriger schwefelsaurer Lösung in $H_2SO_4$ überführt wird und daß die Konzentration des Wasserstoffperoxids und des $SO_2$ so gewählt werden, daß man eine 50 bis 78%ige, vorzugsweise eine 60 bis 75%ige wäßrige Schwefelsäure erhält.

7. Verfahren nach Anspruch 7,
   **dadurch gekennzeichnet,**
   **daß** neben den Sulfaten aus einem MHA-Prozeß Sulfatanteile aus anderen Quellen und/oder Schwefel-haltige Abgase bzw. Abwässer aus einem MHA-Prozeß bzw. anderen Prozessen und/oder organische Abfälle aus einem MHA-Prozeß bzw. anderen Prozessen dem Verbrennungsofen zugeführt werden.

## Claims

1. A process for recovering sulfuric acid from sulfate salts which occur in solution or in solid form in the course of sulfuric hydrolysis of MMP cyanohydrin, wherein the sulfates are converted into $SO_2$ in a combustion furnace,
   **characterised in that**
   the gas mixture containing $SO_2$ is passed through an aqueous sulfuric solution of $H_2O_2$ and the $SO_2$ is converted into sulfuric acid and the concentrations of the hydrogen peroxide and of the $SO_2$ are selected such that a 50 to 78%, preferably a 60 to 75%, aqueous sulfuric acid is obtained.

2. A process according to Claim 1,

**characterised in that**
the concentration of the hydrogen peroxide used is between 10 and 90 wt.%, preferably between 20 and 80 wt. %, particularly preferably between 30 and 60 wt.%.

3. A process according to Claim 1,
**characterised in that**
in the course of the oxidation of $SO_2$ the temperature is between 0 and 100°C, preferably between 10 and 80°C, particularly preferably between 20 and 60°C.

4. A process according to Claim 1,
**characterised in that**
the oxidation of $SO_2$ with $H_2O_2$ is carried out as desired in a one-stage, two-stage or multi-stage apparatus.

5. A process according to Claim 1,
**characterised in that**
in addition to the sulfates from an MHA process, sulfate fractions from other sources and/or sulfurous waste gases or waste waters from an MHA process or other processes and/or organic waste from an MHA process or other processes are supplied to the combustion furnace.

6. A process for the production of 2-hydroxy-4-methylthiobutyric acid (MHA) by hydrolysis of MMP cyanohydrin with sulfuric acid to form a mixture of the principal components MHA and ammonium hydrogensulfate and/or ammonium sulfate, isolation of the MHA, recovery of the sulfuric acid from the separated mixtures containing sulfate and re-use of the sulfuric acid so produced for the hydrolysis of MMP cyanohydrin,
**characterised in that**
the separated mixtures containing sulfate are converted, preferably in the form of an aqueous solution, into $SO_2$ in a combustion furnace at over 800°C, and the gas mixture containing $SO_2$ so produced is converted into $H_2SO_4$ by oxidation with $H_2O_2$ in an aqueous sulfuric solution and the concentrations of the hydrogen peroxide and of the $SO_2$ are selected such that a 50 to 78%, preferably a 60 to 75%, aqueous sulfuric acid is obtained.

7. A process according to Claim 6,
**characterised in that**
in addition to the sulfates from an MHA process, sulfate fractions from other sources and/or sulfurous waste gases or waste waters from an MHA process or other processes and/or organic waste from an MHA process or other processes are supplied to the combustion furnace.

## Revendications

1. Procédé de récupération d'acide sulfurique provenant de sels de sulfate sous forme solide ou par l'hydrolyse sulfatée de cyanhydrine MMP en solution dans laquelle les sulfates sont transformés en $SO_2$ dans un incinérateur, **caractérisé en ce que** le mélange gazeux contenant du $SO_2$ est conduit à travers une solution diluée d'acide sulfurique et de $H_2O_2$, pour être transformé en acide sulfurique et que la concentration de peroxyde d'hydrogène et de $SO_2$ sont choisis de telle manière que l'on obtienne une solution d'acide sulfurique de 50 à 78%, de préférence de 60 à 75%.

2. Procédé selon la revendication 1, **caractérisé en ce que** la concentration du peroxyde d'hydrogène utilisé se situe entre 10 et 90% en poids, de préférence entre 20 et 80% en poids ou de manière particulièrement préférée entre 30 et 60% en poids.

3. Procédé selon la revendication 1, **caractérisé en ce que** la température d'oxydation du $SO_2$ se situe entre 0 et 100°C, de préférence entre 10 et 80°C ou de manière particulièrement préférée entre 20 et 60°C.

4. Procédé selon la revendication 1, **caractérisé en ce que** l'oxydation de $SO_2$ par le $H_2O_2$ est réalisée au choix avec un équipement à une, deux ou plusieurs étapes.

5. Procédé salon la revendication 1, **caractérisé en ce que**, en plus des sulfates provenant d'un processus de MHA, des portions de sulfate provenant d'autres sources et/ou de gaz de combustion ou d'eaux usées contenant du soufre, provenant d'un processus MHA, ou d'autres processus et/ou de déchets organiques, provenant d'un processus MHA, ou d'autres processus sont ajoutées dans l'incinérateur.

6. Procédé de fabrication d'acide 2-hydroxy-4-méthylthioacide butyrique (MHA) par hydrolyse de cyanhydrine MMP avec de l'acide sulfurique en un mélange du composant principal MHA et de l'hydrogénosulfate d'ammonium et/ou de sulfate d'ammonium, par isolation du MHA, par récupération de l'acide sulfurique provenant des mélanges séparés contenant du sulfate et par récupération de l'acide sulfurique ainsi produit pour l'hydrolyse de la cyanhydrine MMP, **caractérisé en ce que** les mélanges séparés contenant du sulfate se présentant de préférence sous la forme d'une solution aqueuse dans un incinérateur sont transformées à une température supérieure à 800°C en $SO_2$, **en ce que** le mélange gazeux contenant du $SO_2$ ainsi produit est transformé en $H_2SO_4$ par oxydation avec le $H_2O_2$ et **en ce que** la concentration de peroxyde d'hydrogène et de $SO_2$ sont choisis de telle manière que l'on obtienne une solution d'acide sulfurique de 50 à 78%, de préférence 60 à 75%.

7. Procédé selon la revendication 6,
**caractérisé en ce que**, en plus des sulfates provenant d'un processus de MHA, des portions de sulfate provenant d'autres sources et/ou de gaz de combustion ou d'eaux usées contenant du soufre provenant d'un processus MHA ou d'autres processus et/ou de déchets organiques provenant d'un processus MHA ou d'autres processus sont ajoutées dans l'incinérateur.

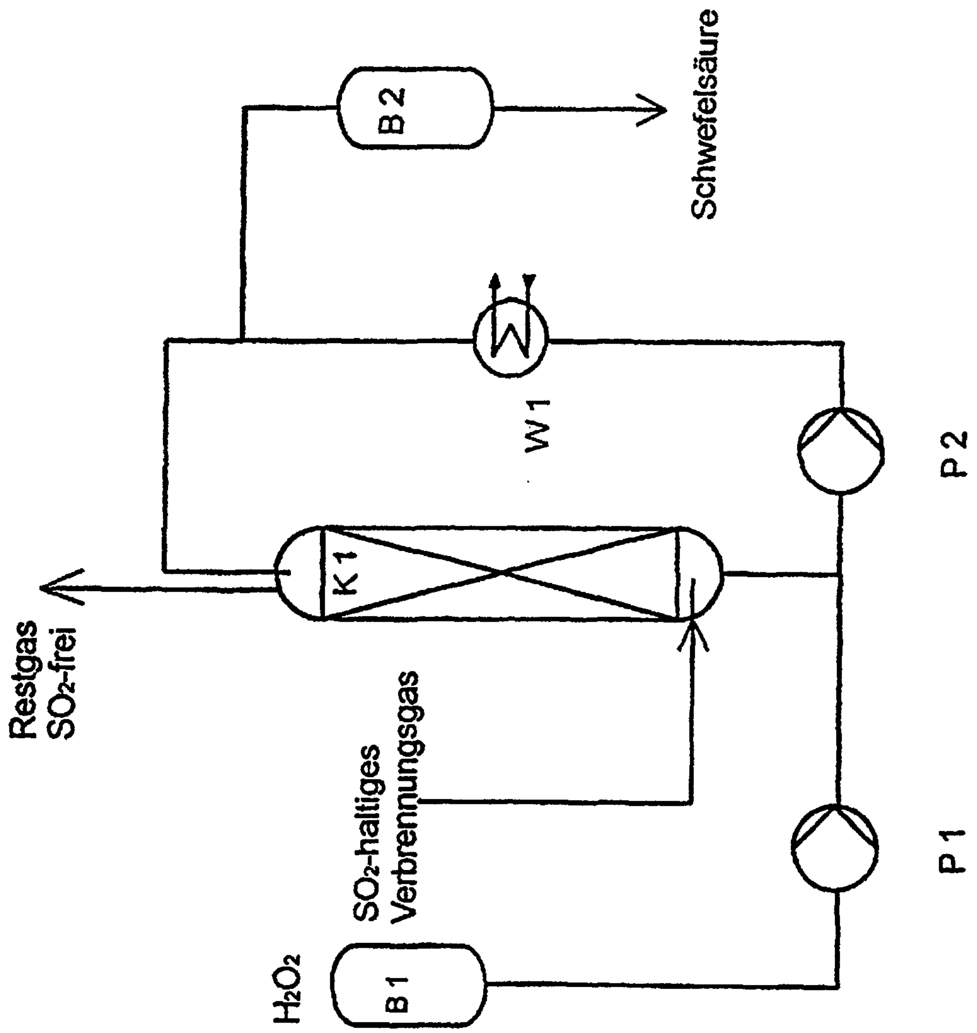
Restgas
SO₂-frei
K 1
$SO_2$-haltiges
Verbrennungsgas
$H_2O_2$
B 1
P 1
P 2
W 1
B 2
Schwefelsäure

Figur 1

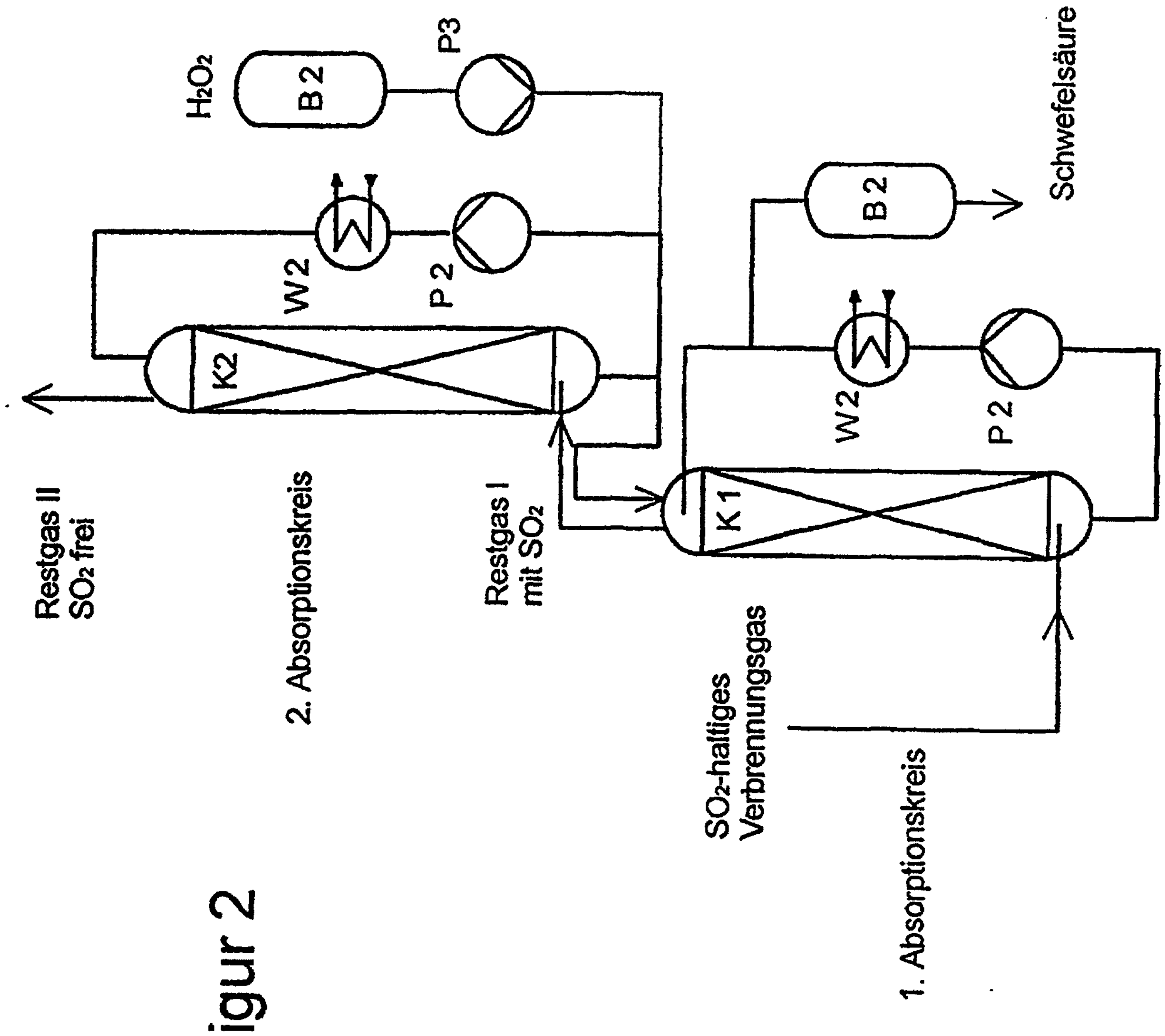
H2O2
B 2
P3
W2
P 2
K2
Restgas II
SO2 frei
2. Absorptionskreis
Restgas I
mit SO2
B 2
Schwefelsäure
W2
P 2
K 1
SO2-haltiges
Verbrennungsgas
1. Absorptionskreis

Figur 2

**Figur 3**

Gesamtverfahren